# EUROPEAN PATENT APPLICATION

(11) **EP 4 685 485 A1**
(43) Date of publication of application: **28.01.2026**
(21) Application number: 25191596.3
(22) Date of filing: 24.07.2025
(51) Int. Cl.: G01N 33/50, C12M 3/06

(54) **APPARATUS AND METHOD FOR APPLYING NORMAL FORCES TO A SUPPORT DEVICE INCLUDING A PERMEABLE MEMBRANE**

(30) Priority: 24.07.2024 US 202463674851 P
(71) Applicant: Southwest Research Institute, San Antonio, Texas 78238-5166 (US)
(72) Inventor: RUBAL, Michael J., Lytle, Texas, 78052 (US); MCMAHON, Nicholas P., San Antonio, Texas, 78254 (US); JARAMILLO, Jasmine L., San Antonio, Texas, 78242 (US)
(74) Representative: Lucke, Andreas

(57) **Abstract**

An apparatus and method for applying normal forces to a support device including a permeable membrane. More specifically, the present invention provides an apparatus and method to apply normal forces to a porous membrane that provides independent access to both sides of the porous membrane layer to evaluate permeation rates of selected drugs through brain blood barrier cells disposed on the membrane.

## Description

This invention was made with government support under Contract Number W15QKN-16-9-1002 awarded by the Defense Threat Reduction Agency. The government has certain rights in the invention.

### FIELD

The present invention stands directed at an apparatus and method for applying normal forces to a support device including a permeable membrane. More specifically, the present invention provides an apparatus and method to apply normal forces to a porous membrane that provides independent access to both sides of the porous membrane layer in order to evaluate permeation rates of selected drugs through brain blood barrier cells disposed on the membrane.

### BACKGROUND

To evaluate the brain blood barrier (BBB) permeability of drug substances, *in-vitro* methods have been developed to retain the most relevant features of the BBB, including relevant cellular based transport mechanisms, for the intention of reliably predicting *in-vivo* brain distribution while also providing relatively affordable methods for screening numerous substances for comparison. Although there have been several designs that have been evaluated, that rely upon the use of porous artificial membrane models to simulate the BBB, one aspect that all the models either fail to address, or address in an inappropriate manner, is the physiological effects associated with the pressure and flow fluctuation of the pulsatile blood flow through the capillaries of the BBB.

In order to enhance permeation rates some membrane models have been placed on orbital shakers, horizontal shakers, or have had magnetic micro-stir bars used to agitate fluid around the membrane. These include membrane models sold as Transwell^{®} BBB *in vitro* model where cells are suspended upon a semi-impermeable membrane insert in a cell culture well, creating upper and lower chambers separated by a cell monolayer. Drug permeability may then be quantified in the Transwell^{®} BBB model by administering a compound to the donor compartment and measuring the compound that has passed into the received compartment, across the "BBB" formed by brain endothelial cells (BEC). However, the directionality of this flow appears horizontal to the membrane.

Accordingly, an on-going need remains to provide improved *in vitro* models of the BBB which are more representative of the *in vivo* BBB, given its role in disease. For example, the pathology of the BBB is important in neurodegenerative diseases such as dementia and Parkinson's disease and understanding the BBB in such diseases may lead to treatment options and improved quality of life.

### SUMMARY

An apparatus and method for *in vitro* determination of permeation rates of a selected drug across blood brain barrier cells supported on a permeable membrane, comprising: (a) providing a support including a permeable membrane wherein said permeable membrane is positioned between upper and lower compartments where said upper and lower compartments contain cell culture media; (b) positioning blood brain barrier cells on the permeable membrane; (c) providing a selected drug in said cell culture media;(d) applying a normal force to said permeable membrane and said blood brain barrier cells on said permeable membrane, wherein said normal force is applied at an angle of 90° +/- 45°; and (e) determining a rate of permeation of said selected drug through said BBB cells and said permeable membrane.

An apparatus for *in vitro* determination of permeation rates of a selected drug across blood brain barrier cells supported on a permeable membrane comprising: (1) a support including a permeable membrane wherein said permeable membrane is positioned between upper and lower compartments wherein said upper and lower compartments contain cell culture media; (2) blood brain barrier cells positioned on the permeable membrane; and (3) a shaker device connected to said support including said permeable membrane wherein said shaker device applies an oscillating normal force of 90° +/- 45° to said permeable membrane and said blood brain barrier cells.

### BRIEF DESCRIPTION OF THE DRAWINGS

**FIG. 1** illustrates a preferred apparatus including a support for a permeable membrane positioned between upper and lower compartments containing cellular media.
**FIG. 2** illustrates the range of normal forces on the permeable membrane and/or BBB cells.
**FIG. 3A** illustrates bEnd3 cells seeded in the control Transwell^{™} support before application of normal forces.
**FIG. 3B** illustrates bEnd3 cells on the Transwell^{™} support after application of normal forces.
**FIG. 3C** illustrates C8-D1A cells seeded on the Transwell^{™} support before application of normal forces.
**FIG. 3D** illustrates C8-D1A cells seed on the Transwell^{™} support after application of normal forces.
**FIG. 4** shows the average measured permeation rates for Formulations 1-5 (Table 1) as a result of the application of normal forces on the permeable membrane.
**FIG. 5** shows the average measured permeation values for pyridostigmine and physostigmine.

### DETAILED DESCRIPTION

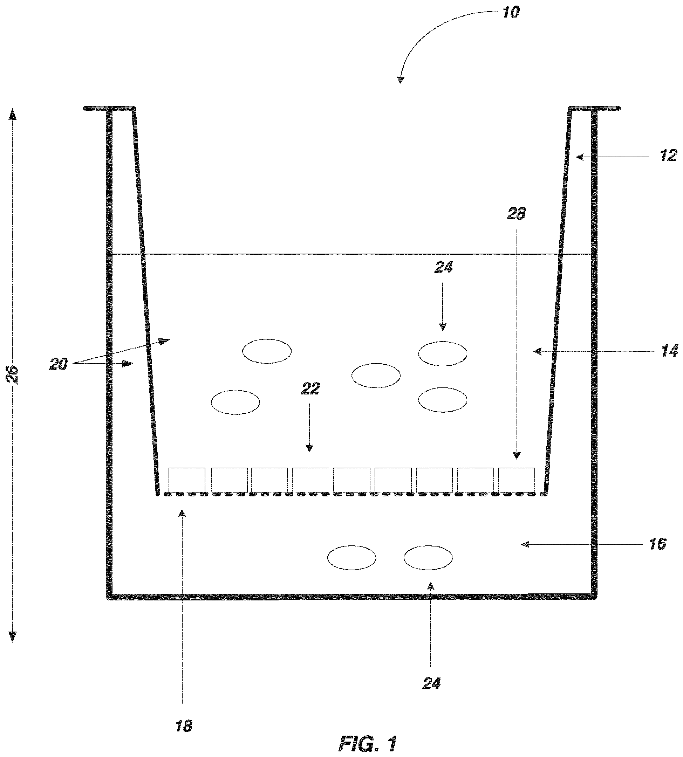

The present invention stands directed at an apparatus and method for applying normal forces to a support device having as a permeable membrane. A preferred example of such an apparatus **10** is illustrated in **FIG. 1****.** The apparatus **10** includes insert or support **12** which is preferably a Transwell^{®} support (available from Corning) which supports a permeable membrane **18** such as a porous membrane. As can also be observed in **FIG. 1** the permeable membrane is positioned in between an upper compartment **14** and lower compartment **16** containing cellular media **20.** BBB cells **22** can then readily disposed on membrane **18.** BBB cells are understood to include endothelial cells. The permeable membrane may preferably be sourced from polyester, polycarbonate or polytetrafluoroethylene/collagen and preferably has a pore size in the range of 0.4 µm to 8.0 µm at a thickness in the range of 5.0 µm to 15.0 µm.

As may be appreciated, the *in vitro* permeability of one or more selected substances **24** (e.g. a drug) through the BBB cells and permeable membrane may be evaluated by, e.g., placement of the substance **24** in the upper compartment and at a selected passage of time (ΔT), the detection and collection of the molecule(s) in the lower compartment can provide a calculation of the molecular permeability through the BBB and permeable membrane **18.** For example, what may be described as a chemoattractant gradient can be developed between the two media filled compartments **14** and **16.** Reference to a drug is reference to any compound that may be utilized to prevent, treat or relieve symptoms of disease or abnormal conditions. In particular, the drugs contemplated for evaluation herein are those targeted for transport across the BBB.

In the present invention, one now applies a generally vertical oscillating force that is preferably illustrated by arrow **26** in **FIG. 1** that creates a normal force (90° +/- 45°) to the membrane **18** and BBB cells **22** positioned on the membrane. See arrow **28.** This oscillating force is preferably provided by a reciprocating shaker device which can be conveniently connected (e.g. clamped) to the device **10.** Such shaker device therefore provides an oscillating normal force (90° +/- 45°) to the permeable membrane and BBB cells disposed thereon. The normal force that is therefore preferably applied is in the range of 0.13 to 5.33 nanonewtons (nN) per 1.0 square micrometer (1.0 µm²) area of the permeable membrane 18 having the BBB cells positioned thereon. This is contemplated to more accurately replicate the actual normal forces that are present on the brain endothelium and therefore provide relatively more accurate *in vitro* modeling.

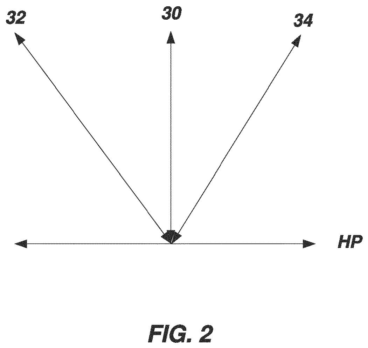

In the broad context of the present invention, and with reference to **FIG. 2****,** the generally vertical oscillating force herein applied to the apparatus **10** is contemplated to provide a normal force on the permeable membrane and BBB cells positioned thereon that intersects a superimposed horizontal plane **("HP")** that aligns with the membrane **18,** at an angle of 90° +/- 45°. This is illustrated by arrow **30** (90° intersect) and arrows **32** (-45°) and **34** (+45 °). As may be appreciated, the control of the imposition of the oscillating force in this manner on apparatus **10** ensures that at least 50% or more of the force imposed on the permeable membrane **18** and/or BBB cells **22** positioned on the membrane can be described herein as a normal force.

Accordingly, the generally vertical oscillating force herein that may be applied to the apparatus **10** having a porous membrane **18** and BBB cells **22** positioned thereon, preferably results in a normal force on the porous membrane and BB cells of 90 ° +/- 45°, or 90° +/- 35°, or 90° +/-25°, or 90° +/- 15°, or 90° +/- 10°, or 90° +/- 5°, or 90° +/- 2.5°. As noted above, this resulting normal force from such generally vertical oscillating force preferably falls in the range 0.13 to 5.33 nanonewtons (nN) per 1.0 square micrometer (1.0 µm²) area of the permeable membrane 18 having the BBB cells positioned thereon.

In this manner, it can now be appreciated that the present invention provides an *in vitro* design to evaluate brain blood barrier permeability of drug substances, that addresses the physiological effects associated with pressure and flow fluctuation of the pulsatile blood flow through the capillaries of the BBB. It is therefore now worth noting that in the arterioles, it has been observed that cyclic stretch and shear stress induced by pulsatile flow can constitute relatively important regulators of vascular endothelial functions, such as proliferation of endothelial and smooth muscle cells, the release of nitric oxide (NO), or the modulation of ATD/ADP conversions. Accordingly, the present invention herein and the application of a generally normal force on the porous membrane in apparatus **10** is contemplated to provide relatively more reliable assay performance by improvement of permeation rates through the membrane and/or membrane selectivity during *in vitro* testing.

Preferably, one may rely upon a vertical shaker to provide the above referenced vertical oscillating force to provide a normal force (90° +/- 45°) to the membrane **18** and BBB cells **22** positioned on the membrane. The vertical shaker preferably applies such vertical oscillating force over an amplitude of 0.1 inches to 6.0 inches, which is the total distance that the vertical shakers travel from peak to trough. For example, if the amplitude is 6.0 inches, the vertical shaker would initially travel up 3.0 inches and then down 3.0 inches. At 0.1 inches, the vertical shaker would initially travel up 0.05 inches and then down 0.5 inches. The vertical shakers also preferably operate at this amplitude and at a frequency of 10 cycles per minute to 300 cycles per minute. In a particularly preferable embodiment, the amplitude for the vertical shakers is a total travel of 0.5 inches to 2.0 inches and a particularly preferred frequency is 60 cycles to 120 cycles per minute.

Computational model permeation results, employing simulation software available from COMSOL, were evaluated to confirm the effect of the normal forces herein on a representative permeable membrane. The parameters were as follows:

| | | |
|---|---|---|
| Size of particles | | 100 nm |
| Porosity of Membrane | | 90% |
| Size of Pores In Membrane | | 1x10⁻⁹ m² |
| Static Conditions | | |
| | Amplitude | 0 m |
| | Frequency | 0 cycles/min |
| Dynamic Conditions | | |
| | Amplitude | 0.70 inches |
| | Frequency | 1.8 Hz |

The computational model indicated that at static conditions, the permeation of the membrane by the particles was at a rate of 1.195 x 10-7 cm/s. Upon application of a normal force due to the dynamic conditions (vertical shaking) noted above, and application of a normal force at 90° to the permeable membrane, the permeation of the membrane by the particles increased to a rate of 1.903 x 10⁻³ cm/s. This confirms a four orders of magnitude increase in permeation rate of the permeable membrane with the applied normal forces as compared to static conditions.

### Working Examples

A reciprocal vertical up and down motion was applied to a Transwell^{®} 24-well permeable support for in-vitro cell testing. The total travel distance for the vertical reciprocating action was about 1.0 inches and the rate was 104 cycles/minute, thereby providing a normal force to the permeable membrane. Computational modeling using software available from COMSOL^{™} confirmed an overall increase in permeations rates for similar sized particles versus an unagitated system.

### Cell Adhesion Testing

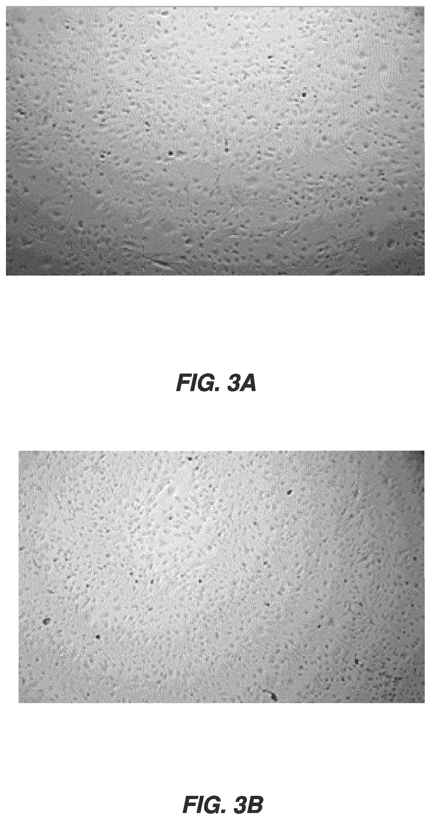

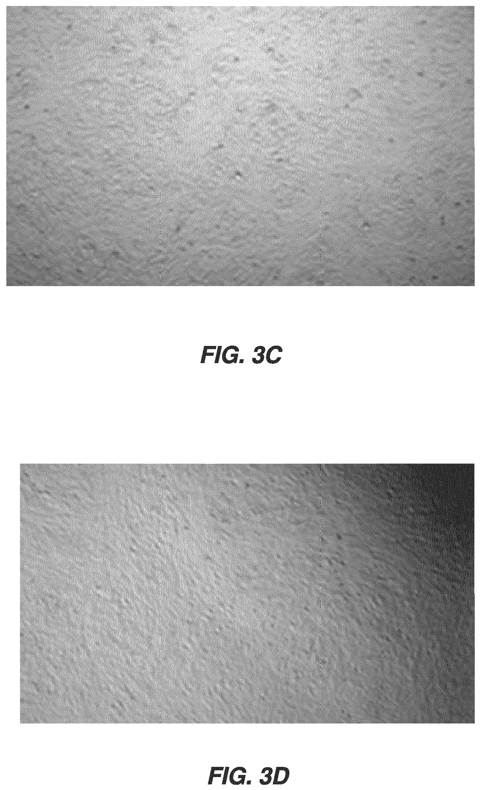

To verify that that the permeable membrane was not disturbed due to normal force agitation the following was conducted. Both C8-D1A and bEnd.3 cells were seeded in a control Transwell^{®} 24-well permeable support for *in-vitro* cellular testing. Once reaching confluence (i.e. when the adherent cells cover the adherent surface of the membrane) the Transwell^{®} 24-well permeable support with the cells was placed in a vertical shaker for 2 hours at 37 °C and 5% CO2 in Hanks Balancing Solution, supplemented with 400 mg/dL of glucose, to ensure that the cells attached and retained their morphology upon application of normal force. C8-D1A and bEnd.3 cell lines remain attached based upon the results shown below in **FIG. 3A, 3B****,** **3C** and **3D****.** Namely, **FIG. 3A** shows the bEnd3 cells seeded in the control Transwell^{™} support before application of normal forces. **FIG. 3B** shows the bEnd3 cells on the Transwell^{™} support after application of normal forces. **FIG. 3C** shows the C8-D1A cells seeded on the Transwell^{™} support before application of normal forces. **FIG. 3D** shows the C8-D1A cells seed on the Transwell^{™} support after application of normal forces. As can be observed, the cells remain attached after the normal force application.

### In Vitro Cellular Based Model Results

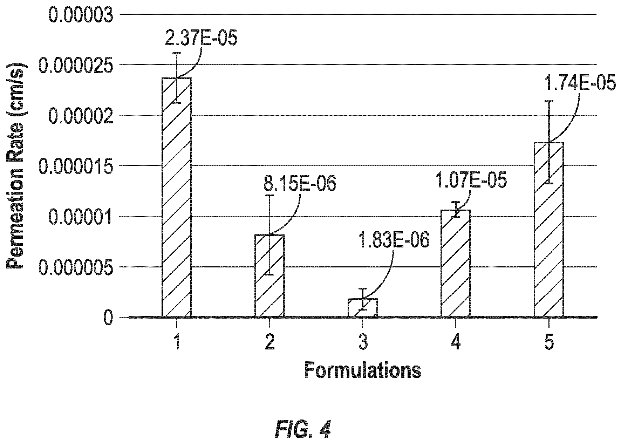

A cell-based *in-vitro* test was used to evaluate multiple fluorescent die containing liposome formulations and identified as Formulation 1, Formulation 2, Formulation 3 Formulation 4 and Formulation 5 in **Table 1** below. Each liposome formulation had a different functionality, but similar size of ~180 nm. A glucose/Hank's balanced salt solution (HBBS) was used as the diluent and the main solution within the acceptor well. The apparatus employed was the apparatus generally illustrated in **FIG. 1****. Table** 1 includes a brief description of the functionality of each formulation for comparison. The average measured permeation values were then plotted in **FIG. 4** along with the min and max error bars with the application of normal force (i.e. a reciprocating action of about 1.0 inches and a rate was 104 cycles/minute for a time period of 2.0 hours.. In **FIG. 4****,** in the absence of application of normal force, the permeation rates were all zero.

From this data a significant threefold (x3) increase in permeation rate was observed from a liposome formulation without transferrin (Formulation 2) to a formulation with transferrin (Formulation 1). It should also be noted that the particle size and zeta potential for both of these samples were very similar and, thus, would not account for such a large difference in performance. This behavior is consistent with known use of transferrin as a cellular based transport.

The addition of a positively charged phospholipid into Formulation 3 dramatically reduced the apparent permeation rate. This is an interesting finding, as the positive charge of the liposomes and the negative nature of the cellular membrane should encourage liposome attraction to the membrane and has been used in the literature to aid in selectivity of liposomes to the BBB. However, it is quite possible that this electrostatic charge interaction is in effect here, but inhibits liposome permeation by adherence of the liposomes to the membrane instead of permeation through the membrane.

Formulation 4 with both the positive phospholipid and transferrin, seems to overcome the binding effect of the positive phospholipids, and restore the permeation rates comparable to Formulation 2 which does not have a positive phospholipid component nor the transferrin. It should be noted that a change in zeta potential is noticed during the final step in Formulation 4. Prior to addition of transferrin, the zeta potential was a positive 3.0 mV. After addition of transferrin, the zeta potential became a negative -2.8 mV.

Finally, for Formulation 5, the addition of twice the amount of transferrin sites on the liposomes compared to Formulation 1, still had an increase in permeation rates compared to Formulation 2 (without transferrin), but was slightly less than what was observed Formulation 1. Therefore, no additional benefit was observed for additional amounts of transferrin.

**Table 1**

| **Formulation** | **Functional Groups Present** | | | | **Zeta Potential** |
|---|---|---|---|---|---|
| | **part mol to PL** | | | **Size** | |
| | **PL-PEG** | **PL+** | **PL-PEG-TF** | **nm** | **mv** |
| 1 | 1 | 0 | 0.1 | 175.0 | -1.9 |
| 2 | 1 | 0 | 0 | 182.8 | -2.1 |
| 3 | 1 | 10 | 0 | 164.0 | 0.7 |
| 4 | 1 | 10 | 0.2 | 199.3 | -2.8 |
| 5 | 1 | 0 | 0.2 | 185.0 | -5.6 |

To further test the cell-based *in-vitro* BBB assay, two compounds were selected as known controls that cross the blood brain barrier. Namely, physostigmine and pyridostigmine were selected as known controls for testing a cell in the *in vitro* blood-brain barrier (BBB) model due to their well-documented ability to cross the BBB. Physostigmine, a reversible cholinesterase inhibitor, is notable for its capacity to readily penetrate the central nervous system, making it a suitable candidate for evaluating the permeability of the BBB model. Pyridostigmine, although primarily used in the treatment of myasthenia gravis, also demonstrates the ability to cross the BBB, albeit to a lesser extent than physostigmine. By utilizing these compounds as controls, the integrity and functionality of the *in vitro* BBB model can be effectively assessed, ensuring its reliability for further studies. Accordingly, both physostigmine and pyridostigmine were placed in the apparatus of **FIG. 1** along with the permeable membrane containing BBB cells (both C8-D1A and bEnd.3 cells) on the porous membrane and the apparatus was subject to a reciprocating action of about 1.0 inches and a rate was 104 cycles/minute for a time of 2.0 hours.

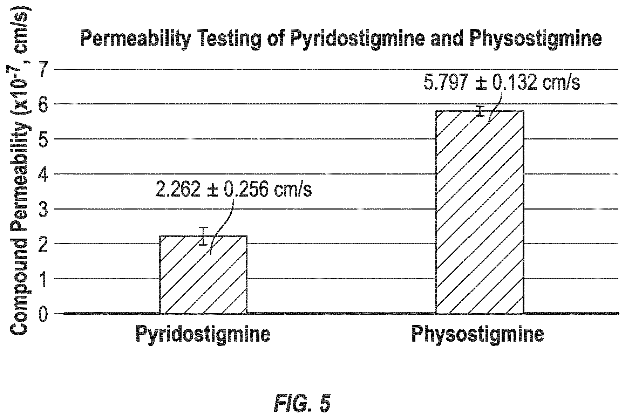

The average measured permeation values were plotted in **FIG. 5** along with the min and max error bars. The permeation rates show the ability to cross the BBB with the expected differences between physostigmine (5.79749 x 10⁻⁷ cm/s) and pyridostigmine (2.26157 x 10⁻⁷ cm/s) of crossing ability.

As can therefore be seen, the present invention provides both an apparatus and method for applying normal forces to a permeable support device, such as a Transwell^{™} permeable support, to evaluate in vitro permeability of a selected molecule (e.g. a drug). The application of normal forces to the permeable membrane in the support device, containing BBB cells as a membrane supported by the porous membrane, does not disrupt the BBB membrane and exhibits permeation rates that appear acceptable for commercial drug bearing formulations as well as acceptable permeation rates from pyridostigmine and physostigmine controls.

## Claims

1. An *in vitro* method of determining permeation rates of a selected drug across blood brain barrier cells supported on a permeable membrane, comprising:
a. providing a support including a permeable membrane wherein said permeable membrane is positioned between upper and lower compartments where said upper and lower compartments contain cell culture media;
b. positioning blood brain barrier cells on the permeable membrane;
c. providing a selected drug in said cell culture media;
d. applying a normal force to said permeable membrane and said blood brain barrier cells on said permeable membrane, wherein said normal force is applied at an angle of 90° +/- 45°; and
e. determining a rate of permeation of said selected drug through said BBB cells and said permeable membrane.

2. The method of claim 1, wherein said normal force is in the range of 0.13 to 5.33 nanonewtons per 1.0 µm² area of said permeable membrane having the blood brain barrier cells positioned thereon.

3. The method of claim 1, wherein said normal force is applied by application of a vertical reciprocating action on said permeable membrane and said BBB cells.

4. The method of claim 1, wherein said permeable membrane includes pores at a size in the range of 0.4 µm to 8.0 µm and at a thickness in the range of 5.0 µm to 15.0 µm.

5. The method of claim 1, wherein said normal force is applied at an angle of 90° +/- 35°.

6. The method of claim 1, wherein said normal force is applied at an angle of 90° +/- 25°.

7. The method of claim 1, wherein said normal force is applied at an angle of 90° +/- 15°.

8. The method of claim 1, wherein said normal force is applied at an angle of 90° +/- 10°.

9. The method of claim 1, wherein said normal force is applied at an angle of 90° +/- 5°.

10. The method of claim 1, wherein said normal force is applied at an angle of 90° +/- 2.5°.

11. The method of claim 1, wherein said normal force is applied by a vertical oscillating shaker over an amplitude of 0.1 inches to 6.0 inches at a frequency of 10 cycles per minute to 300 cycles per minute.

12. An apparatus for *in vitro* determination of permeation rates of a selected drug across blood brain barrier cells supported on a permeable membrane comprising:
a. a support including a permeable membrane wherein said permeable membrane is positioned between upper and lower compartments wherein said upper and lower compartments contain cell culture media;
b. blood brain barrier cells positioned on the permeable membrane;
c. a shaker device connected to said support including said permeable membrane wherein said shaker device applies an oscillating normal force of 90° +/- 45° to said permeable membrane and said blood brain barrier cells.

13. The apparatus of claim 12, wherein said shaker device applies an oscillating normal force in the range of 0.13 to 5.33 nanonewtons per 1.0 µm² area of said permeable membrane having the blood brain barrier cells positioned thereon.

14. The apparatus of claim 12, wherein said permeable membrane includes pores at a size in the range of 0.4 µm to 8.0 µm and at a thickness in the range of 5.0 µm to 15.0 µm.

15. The apparatus of claim 12, wherein said shaker device applies a normal force at an angle of 90° +/- 35°.

16. The apparatus of claim 12, wherein said shaker device applies a normal force at an angle of 90° +/- 25°.

17. The apparatus of claim 12, wherein said shaker device applies a normal force at an angle of 90° +/- 15°.

18. The apparatus of claim 12, wherein said shaker device applies a normal force at an angle of 90° +/- 10°.

19. The apparatus of claim 12, wherein said shaker device applies a normal force at an angle of 90° +/- 5°.

20. The apparatus of claim 12, wherein said shaker device applies a normal force at an angle of 90° +/- 2.5°.

21. The apparatus of claim 12, wherein said normal force applied by said shaker device is applied over an amplitude of 0.1 inches to 6.0 inches at a frequency of 10 cycles per minute to 300 cycles per minute.
